# NEW EUROPEAN PATENT SPECIFICATION

(11) **EP 0 706 364 B2**
(45) Date of publication and mention of the opposition decision: **21.03.2001**
(45) Mention of the grant of the patent: 04.02.1998
(21) Application number: 94920619.7
(22) Date of filing: 22.06.1994
(51) Int. Cl.: A61F 13/36, A61F 13/15

(54) **A METHOD OF MANUFACTURING ABSORBENT ARTICLES, SUCH AS ABSORBENT TOWELS, SURGICAL TOWELS OR THE LIKE, AND AN ARTICLE MANUFACTURED IN ACCORDANCE WITH THE METHOD**
VERFAHREN ZUM HERSTELLEN ABSORBIERENDER ARTIKEL, WIE ETWA ABSORBIERENDE TÜCHER,CHIRURGISCHE TÜCHER ODER ÄHNLICHES UND EIN ARTIKEL DER NACH DIESEM VERFAHREN HERGESTELLT WIRD
PROCEDE DE FABRICATION D'ARTICLES ABSORBANTS TELS QUE SERVIETTES, SERVIETTES A USAGE CHIRURGICAL ET AUTRES ET ARTICLES ASSOCIES

(30) Priority: 28.06.1993 SE 9302224
(43) Date of publication of application: 17.04.1996
(73) Proprietor: Mölnlycke AB, 405 03 Göteborg (SE)
(72) Inventor: FABO, Thomas, S-435 41 Mölnlycke (SE); SOLMEUS, Sune, S-435 43 Mölnlycke (SE); JOHANNISON, Ulf, S-438 93 Landvetter (SE); KUUSE, Staffan, S-430 63 Hindas (SE); NILSSON, Lennart, S-436 55 Hovas (SE)
(74) Representative: Hyltner, Jan-Olof
(86) International application number: SE9400624
(87) International publication number: WO9500097

(56) References cited:
- DE-A- 3 002 136
- GB-A- 1 061 054
- US-A- 4 489 118
- US-A- 4 578 070
- US-A- 4 726 977

## Description

The present invention relates to a method of manufacturing articles for absorbing blood or other wound fluids, such as compresses, surgical towels and like articles, and to an article manufactured in accordance with the method.

US-A-4 578 070 discloses such an article.

Surgical towels known for example from US 4,075,382, so-called abdominal towels, are used for many different purposes in operative surgery. For instance, such towels are used to prevent blood flowing into the area of the operation wound in the case of abdominal operations, preventing leakage of blood past the region of the operation wound, to mop-up blood that has leaked past the wound, and so on. Towels of this kind shall thus be effectively absorbent, easy to bring to a desired shape and readily handled in general. Since a large number of surgical towels are used in operative surgery, they should also be simple and cheap to manufacture.

Surgical towels which are comprised of two or more gauze layers that are joined together by a circumferentially extending seam fulfil the aforesaid requirements to a large extent, but have the drawback of containing a high proportion of fuzz or fluff, in other words release too many loose fibres or "linters" that can fasten in wounds or tissue, and are complicated in manufacture due to the fact that it is necessary to sew the gauze layers together. To avoid these drawbacks, surgical towels made of nonwoven fabric and having a low fuzz content have been proposed, and it is also known in the present context to enclose a layer of material which has a high fuzz content between two layers of material of low fuzz content, in order to obtain a product of low total fuzz content. One drawback with the use of nonwoven material is that the material has a tendency to slide against tissue and therewith move from its intended position in a wound area, for instance. Nonwoven materials are also difficult to grip and are relatively thin and therewith have a relatively low absorbance capacity per unit of area. This means that it may be necessary to use several mutually joined nonwoven layers in order to obtain the desired absorbency per unit area of the manufactured surgical towel, leading to relatively complicated and expensive manufacture.

The object of the present invention is to provide a simple and cost-favourable method of manufacturing articles of the aforesaid kind which satisfy high functional requirements.

This object is achieved in accordance with the invention with a method according to Claim 1.

According to one embodiment of the invention, there are used webs of material which each include an absorbent first layer which is wrinkled permanently over the whole of its surface, and a smooth second layer of thermoplastic material which is fastened to the first layer; wherein the two webs are joined together along the contours of the articles to be manufactured and the articles to be manufactured are cut from the thus combined webs along the join lines. Joining of the webs and cutting of finished articles is effected preferably in one single operation and with the aid of ultrasound welding.

According to one variant of the preferred embodiment, the article is provided with a hanging strap when joining the webs together and cutting-out the finished articles. Each first layer of the webs is permanently wrinkled by advancing the first layer to an ultrasound welding device at a higher speed than the speed of the layer to which said first layer shall be fastened, and by joining the layers together with the aid of a pattern of discrete fastening points which extend across the entire surface of the first layer.

According to another variant of the inventive method, a third web of absorbent material is introduced in between the mutually facing smooth layers of the first and second webs prior to joining the webs together.

The invention also relates to an article according to Claim 9.

According to one embodiment, the article includes two smooth second layers.

According to a preferred embodiment, the article includes a hanging strap which is formed integrally with the remainder of the article, and the first layer is comprised of two-layer material.

The invention will now be described in more detail with reference to the accompanying drawings, in which
Fig. 1 illustrates schematically a plant for manufacturing articles in accordance with one embodiment of the invention;
Figs. 2-5 illustrate different ways of cutting-out surgical towels;
Fig. 6 is a perspective view of an inventive surgical towel;
Fig. 7 is a sectional view taken on the line V-V in Figure 6; and
Fig. 8 is a schematic view of a plant for manufacturing articles in accordance with a second, preferred embodiment of the invention.

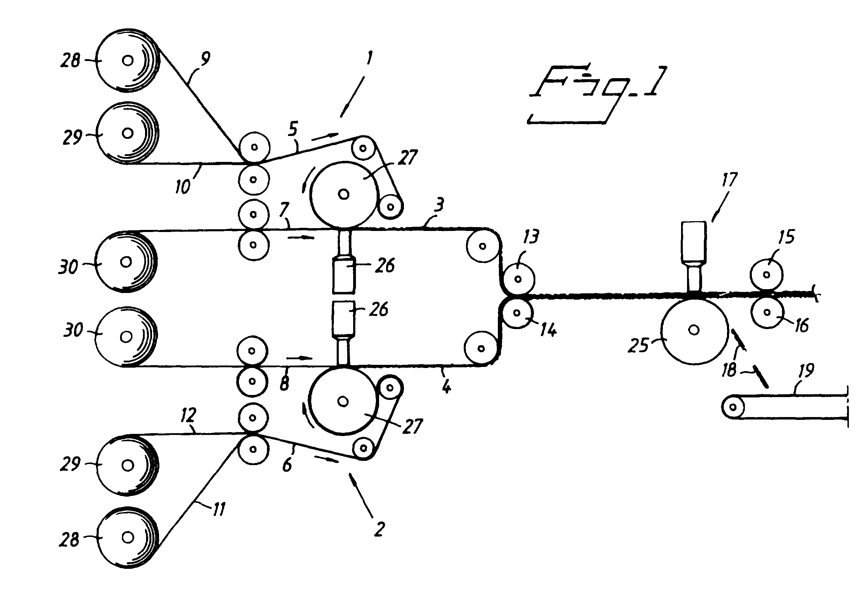

Figure 1 illustrates a surgical towel manufacturing plant which includes two identical plant arrangements 1, 2 which function to produce a respective web of material 3 and 4. The webs are comprised of a wrinkled, absorbent first layer 5 and 6 respectively, which are fastened to a respective smooth second layer 7 and 8. In the illustrated case, the first layers 5, 6 are comprised of two material layers 9, 10 and 11, 12 respectively.

The plant also includes means for combining the webs 3, 4 with their smooth layers 7 and 8 facing one another. These means are comprised of feed rollers 13, 14, which together with feed rollers 15, 16 advance the combined webs 3, 4 through a device 17 which functions to join the webs together and to cut surgical towels therefrom. The surgical towels cut from the webs are then passed to a station (not shown) on a conveyer 19 for instance, where the towels are sterile-packaged.

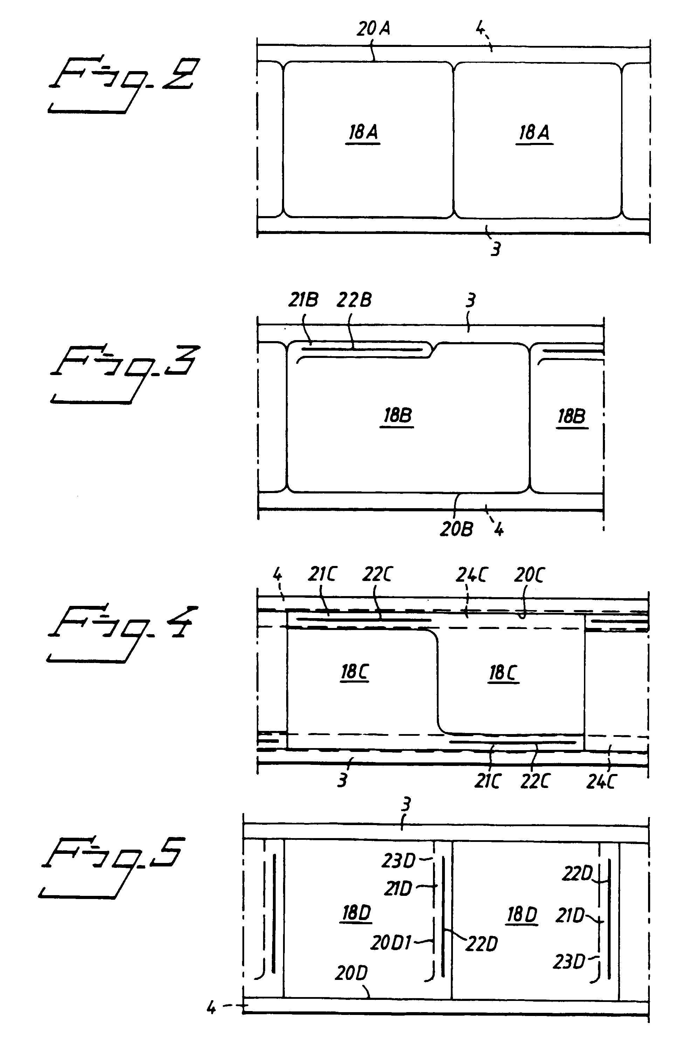

Figures 2-5 illustrate from above different ways in which surgical towels 18A-18D are cut from the webs 3, 4 in the device 17.

Figure 2 illustrates a pattern of cutting lines 20A with the aid of which rectangular surgical towels 18A with rounded corners are cut from the webs 3, 4.

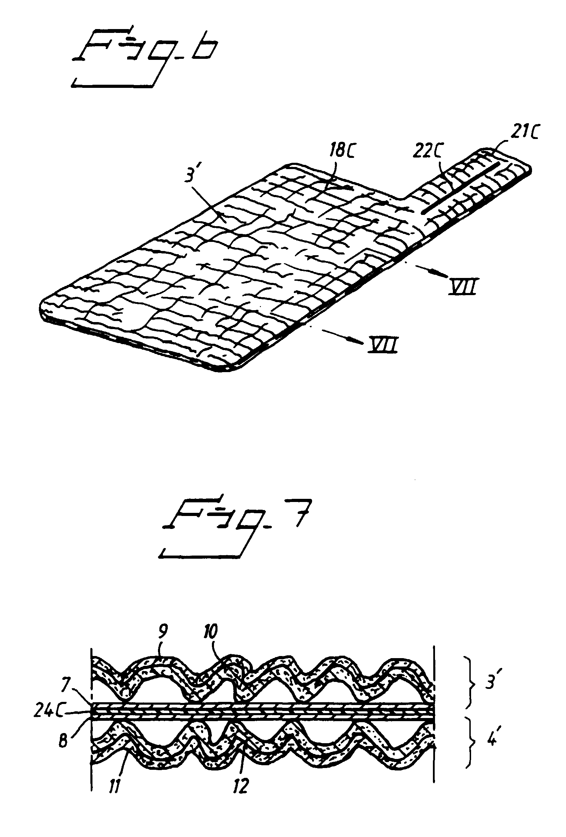

In the Figure 3 illustration, rectangular surgical towels 18B are cut-out along the cutting lines 20B. These towels differ from the towels 18A, in that hanging straps 21 B are also cut-out from the webs 3, 4. The straps are provided with a slot 22B so as to enable them to be hung on a hook or like device.

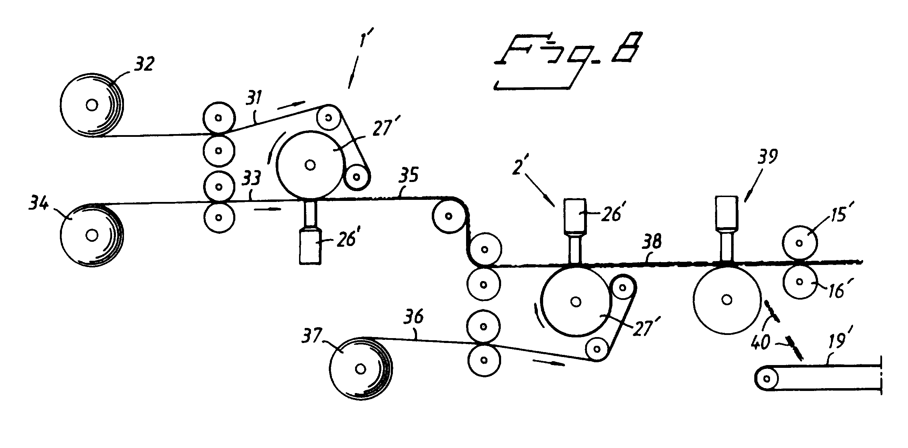

Figure 4 illustrates a variant of the inventive method of an inventive surgical towel, in which a strip 24C of textile material, non-woven material, or plastic material is placed between the edges of the towel cut-outs that extend in the machine direction. These strips are conveniently placed between said edges when bringing the webs 3, 4 together, with the aid of the rollers 13, 14. The cutting lines 20C also define in this case a generally rectangular surgical towel 18C including a hanging strap 21C which constitutes an extension of the towel 18C edge-parts extending in the machine direction. A slit 22C is cut from respective hanging straps 21C, and the strip 24C reinforces the strap. It will be understood that a strengthening strip may also be laid in the strap 21 B of the surgical towel 18B shown in Figure 3.

Figure 5 shows another example of how a hanging strap 21D provided with a slit 22D can be cut from the surgical towels 18D cut from the webs 3, 4, along cutting lines 20D. In addition to the position of the strap 21 D on the surgical towel 18D, this example also differs from the earlier described examples by virtue of breaks or interruptions 23D in the strap cutting line 20D1 facing towards the remainder of the towel. As a result, the straps 21 D will remain appended to the remainder of an associated surgical towel after cutting the towels 18D from the webs. This gives the user the choice of allowing the strap to remain attached to the towel, or of tearing-off the strap, whichever is desired. It will be understood that the surgical towels 18B and 18C can also be provided with detachable straps in a similar way.

The device 17 is preferably comprised of an ultrasound welding device comprising an anvil roller 25 which includes radially and outwardly projecting anvils configured so that welding together of the webs 3, 4 and cutting of the surgical towels 18 therefrom will be effected in one single operation. This can be achieved with anvils having anvil surfaces that are located at mutually different levels, wherein the radially outer surface forms a cutting surface, while the radially inner surface forms a welding surface. These surfaces may merge continuously with one another. A device 17 of this construction will enable the webs 3, 4 to be joined together and surgical towels cut therefrom in a particularly simple fashion, with the aid of only a few components. Furthermore, a device 17 constructed in this manner will result in soft joins in the product cut-out.

The identical arrangements 1, 2 functioning to produce the webs 3, 4 are constructed in the manner described in Swedish Patent Application No. 9202993-3 filed on the 12th October 1992 and to which reference is made for further details of the construction of these arrangements. It suffices to mention in the instant application that two layers 5, 7 and 6, 8 respectively are fed in between the anvil roller 27 and the ultrasonic horn 26, the layers 5, 6 being advanced at a higher speed than the layers 7, 8. This results in wrinkles or pleats in the layers 5, 6, which are made permanent when the layers 5 and 7 and the layers 6 and 8 respectively are bonded together by means of discrete fastening points, which extend over the complete surface of the surface in a regular pattern. The term "fastening points" as used here is also meant to include complete areas that are joined in a punctiform fashion, such as elongated connecting regions for instance, although the "fastening point" shall have a small extension in relation to the dimensions of the finished article. As will also be seen from Figure 1, the layers 5, 6 are comprised of two mutually joined layers 9, 10 and 11, 12 respectively, which are taken from storage reels 28, 29. The layers 7, 8 are taken from storage reels 30.

The invention thus provides a method whereby surgical towels can be produced simply and in a cost-favourable manner in a continuous process from smooth material wound on storage reels.

Figures 6 and 7 illustrate a surgical towel 18C which has been manufactured by means of the aforedescribed method and cut-out in accordance with the pattern shown in Figure 4. The surgical towel 18C thus comprises two identical pieces 3', 4' of the three-layer webs 3, 4 having two wrinkled layers 9, 10 and 11, 12 respectively and a smooth layer 7 and 8 respectively. The smooth layers face one another and the three-layer pieces 3', 4' are joined together around their peripheral edges and on both sides of the slit 22C. As will be seen from the cross-sectional view in Figure 6, the hanging strap 21C is reinforced with a strip 24C, for instance a plastic strip, which extends along that edge part of the towel 18C of which the strap is an extension.

The smooth layers 7 and 8 shall consist of a weldable material, and are preferably comprised of non-woven polypropylene fibres. In addition to enabling the webs 3, 4 to be welded together, the layers are also intended to take-up tensile forces in the material pieces 3', 4', so as to prevent the wrinkles in the layers 9, 10 and 11, 12 respectively from being smoothed-out as a result of such tensile forces. The layers 7 and 8 have a surface weight of 10-20 g/m².

The layers 10 and 12 shall be absorbent and are preferably comprised of a nonwoven material which includes absorbent fibres, for instance viscose fibres. These layers may advantageously comprise spunlace material. The layers may also include non-absorbent fibres, for instance polyester fibres, so as to impart to the layers sufficient strength to enable them to withstand the wrinkling and handling processes to which they are subjected. The percentage of absorbent fibres present, however, should not be less than 50%. The layers 10 and 12 have a surface weight of 20-100 g/m². In addition to absorbing liquid the layers 10 and 12 also have the purpose of quickly dispersing absorbed fluid and to impart a relatively high bulk to the composite surgical towel. The towel has a thickness of between 3-10 mm, said thickness primarily depending on the size of the wrinkles.

As with the layers 7 and 8, the layers 9 and 11 shall also be made of a weldable material, such as a nonwoven material, for instance comprised of polypropylene fibres. These layers must also be liquid-permeable and the fibres in the layers 9, 11 may be treated with an agent which renders them hydrophilic, so as to facilitate the liquid absorbency of the layers 10 and 12. These layers also form the outer surface layers of the surgical towel and shall therefore have very little fuzz, i.e. the fibres in the layers 9 and 12 shall be well bonded to one another so that only some or a very few fibres will loosen from the layers when the towel is used. Accordingly, the layers 9 and 11 may conveniently be comprised of spun-bonded nonwoven material. The surfaces 9 and 11 have a surface weight of between 10-20 g/m².

Figure 8 illustrates schematically a plant for manufacturing surgical towels in accordance with a second, preferred embodiment of the invention. Similar to the plant illustrated in Figure 1, the plant illustrated in Figure 2 includes two identical plant arrangements 1', 2' which function to wrinkle a layer of material and fasten this layer to another layer, said arrangements including an ultrasonic horn 26' and anvil rolls 27'. A first layer 31 is fed from a storage reel 32 through the arrangement 1' at a higher speed than the speed of a second layer 33 taken from a storage reel 34, and the layers are welded together as they pass the ultrasonic horn 26' of the arrangement 1' to form a web 35 having a first, permanently wrinkled layer and a smooth second layer. The web 35 is then advanced as a second layer through the arrangement 2' and is there joined to a first layer 36 taken from a storage reel 37, this first layer being wrinkled and fastened to the smooth undersurface of the composite web 35. In this way, there is produced a three-layer material web 38 having two outer, permanently wrinkled layers 31, 36 and a smooth intermediate layer 33. The three-layer web 38 is then passed through an arrangement 39 in which individual surgical towels 40 are cut from the web. In this Figure 8 embodiment, the arrangement 39 has the form of an ultrasonic arrangement, although other types of cutting tools may, of course, be used instead. The surgical towels 40 cut from the web are then transported on a conveyor 19' for packaging and sterilization. As in the case of the plant illustrated in Figure 1, the various layers are advanced through the plant with the aid of feeder rolls, for instance the feeder rolls 15', 16' shown in Figure 8. In the case of the Figure 8 embodiment, the wrinkled layers 31, 36 are comprised of single-layer material. The two outer, wrinkled layers are comprised of spun lace, nonwoven material, similar to the absorbent layers 10, 12 of the Figure 1 embodiment, although they include at least 10% weldable fibres, for instance polypropolene fibres.

The described surgical towels are less expensive to produce than multi-layer surgical towels made of textile or nonwoven material that must be sewn and twisted or hemmed, in particular when a separate hanging strap shall be sewn onto the towels. The inventive towels also have functional advantages over known surgical towels made of textile material or nonwoven material. One advantage is that the towels can be given well-bonded outer surface layers, which provides a surgical towel which has very little fuzz or "linters". Another advantage is that the wrinkles in the two outermost layers provide greater friction against contiguous material when using the towel than with a smooth nonwoven layer, which enables smooth organs to be held firmly more readily by the surgical towel and facilitates "mopping-up" of particularly highly viscous liquids. Wrinkling of the surfaces also gives the material a greater bulk, which enables the material to be gripped and handled more easily. Another advantage afforded by the described surgical towel over towels made of textile material is that the inventive towel will not collapse or lose its stability in a wet state, as will gauze towel or pad.

It will also be understood that the described embodiments can be modified in several ways within the scope of the invention. For instance, more than two webs can be combined to form a composite structure from which products are cut when thicker towels are required, for instance thicker surgical towels, pads, wads or compresses. Furthermore, the wrinkled layers may be comprised of more than two layers. The towel cut-outs may be folded-out and then folded to form thicker towels, and other fibres having similar properties can be used instead of the aforesaid fibre types. Instead of performing the wrinkling, combining and cutting-out steps in one continuous process on the basis of component layers rolled on storage reels, the inventive method can be carried out with premanufactured webs which include a smooth layer and one or more wrinkled layers and which are rolled onto storage reels. The web combining and web cutting features of the method may be carried out in two consecutive steps instead of in one single step as in the described embodiment. If it is wished to avoid the wastage occasioned by the edge parts that remain on the web when cutting in accordance with the patterns illustrated in Figures 2-5, the mutually combined webs can be passed through the web combining and web cutting device with the aid of suitable conveyers. An absorbent layer of fluff pulp for instance can be inserted between the webs prior to bringing said webs together. The invention is therefore limited solely by the content of the following claims.

## Claims

1. A method of manufacturing compresses, towels, surgical towels or like articles for absorbing blood and other wound fluids, by mutually combining a first and a second web of material (3, 4; 35, 36), of which at least one web includes an absorbent first layer (5; 31) which is permanently wrinkled over the whole of its surface, and a smooth, second layer (7; 33) of thermoplastic material fastened to the first layer, and of which the second web at least includes a first layer (6; 36) which is wrinkled over the whole of its surface, wherein said first and second webs are combined with the wrinkled first layers (5, 6; 31, 36) facing away from one another, the wrinkles in the respective first layer being made and made permanent by fastening the first layer to a smooth second layer of thermoplastic material, each first layer being joined to a smooth second layer of thermoplastic material by means of a pattern of discrete fastening points which extends over the whole surface of the first layer; and by mutually joining the two webs of material (3, 4; 35, 36) and cutting the articles (18; 40) to be manufactured from the thus joined webs.

2. A method according to Claim 1, **characterized** by using webs of material (3, 4) which each include an absorbent first layer (5, 6) which is permanently wrinkled over the whole of its surface, and a smooth second layer of thermoplastic material (7, 8) which is fastened to the first layer; joining the two webs together along the contours of the articles (18) to be manufactured; and cutting the articles from the thus joined webs in the join lines.

3. A method according to Claim 2, **characterized** by joining the webs (3, 4) together and cutting the finished articles (18) from the webs in one single operation.

4. A method according to Claim 3, **characterized** by joining the webs together and cutting the articles therefrom with the aid of an ultrasound welding process.

5. A method according to any one of Claims 1-4, **characterized** by forming a hanging strap (21 B-21 D) on the article (18B-18D) when joining the webs (3, 4) together and cutting-out finished articles.

6. A method according to Claim 5, **characterized** by forming the hanging strap (21B-21C) in one of the edges of the article (18B-18C9) extending longitudinally in the machine direction; and placing a reinforcing strip (24C) between the webs (3, 4) so that said strip extends in the region of the hanging strap of each article.

7. A method according to any one of the preceding Claims, **characterized** by feeding the first layer (5, 6) to an ultrasound welding device (26, 27) at a speed greater than the speed of the layer (7, 8) to which the first layer is to be fastened, so as to permanently wrinkle each of the first layers of the material webs; and joining the layers together by means of a pattern of discrete fastening points which extend over the whole surface of the first layer.

8. A method according to any one of the preceding Claims, **characterized** by feeding a third web of absorbent material in between the mutually facing smooth layers of the first and the second webs prior to joining said webs together.

9. An article for absorbing blood and other wound fluids, such as a compress, towel or surgical towel manufactured by the method according to Claim 1, wherein that the article includes two absorbent first layers (10, 12; 31, 36) of nonwoven material which are wrinkled permanently over the whole of their respective surfaces, and at least one smooth, second layer (7, 8; 33) of thermoplastic material, said layers being joined together each first layer being joined to a smooth second layer of thermoplastic material by means of a pattern of discrete fastening points which extends over the whole surface of the first layer, the at least one second layer being placed between the first layers.

10. An article according to Claim 9, **characterized** in that the article includes two smooth second layers (7, 8).

11. An article according to Claim 9 or 10, **characterized** in that the article includes a hanging strap (21B-21D), which is formed integrally with the article (18B-18D).

12. An article according to any one of Claims 9-11, **characterized** in that the first layer (5, 6) is comprised of a two-layer material (9, 10 and 11, 12 respectively).

## Patentansprüche

1. Verfahren zur Herstellung von Kompressen, Tüchern, chirurgischen Tüchern oder ähnlichen Artikeln zum Absorbieren von Blut und anderen Wundflüssigkeiten, durch Überlagern einer ersten und einer zweiten Materialbahn (3, 4; 35, 36), von denen zumindest eine Bahn eine absorbierende erste Schicht (5; 31), die über die gesamte Oberfläche dauerhaft geknittert ist, und eine glatte zweite Schicht (7; 33) aus thermoplastischem Material beinhaltet, die an der ersten Schicht angebracht ist, und von denen die zweite Bahn zumindest eine erste Schicht (6; 36) beinhaltet, die über ihre gesamte Oberfläche geknittert ist, wobei die erste und die zweite Bahn einander überlagert werden, so dass die geknitterten ersten Schichten (5, 6; 31, 36) voneinander weg zeigen; wobei die Knittstellen in der jeweiligen ersten Schicht erzeugt und dauerhaft gemacht werden, indem die erste Schicht an der glatten zweiten Schicht aus thermoplastischem Material angebracht wird, und wobei jede erste Schicht mit einer glatten zweiten Schicht aus thermoplastischem Material durch ein Muster aus diskreten Befestigungspunkten verbunden ist, das sich über die gesamte Oberfläche der ersten Schicht erstreckt und durch miteinander Verbinden der zwei Materialbahnen (3, 4; 35, 36) und Schneiden der herzustellenden Artikel (18; 40) aus den so verbundenen Bahnen.

2. Verfahren nach Anspruch 1, **gekennzeichnet durch** Verwendung von Materialbahnen (3, 4), die jeweils eine absorbierende erste Schicht (5, 6) beinhaltet, die über ihre gesamte Oberfläche beständig geknittert ist, und eine glatte zweite Schicht aus thermoplastischem Materials (7, 8) beinhaltet, die an der ersten Schicht angebracht ist; miteinander Verbinden der zwei Bahnen entlang der Konturen der herzustellenden Artikel (18); und Ausschneiden der Artikel aus den derart verbundenen Bahnen an den Verbindungslinien.

3. Verfahren nach Anspruch 2, **gekennzeichnet durch** miteinander Verbinden der Bahnen (3, 4) und Ausschneiden der fertigen Artikel (18) aus den Bahnen in einem einzigen Vorgang.

4. Verfahren nach Anspruch 3, **gekennzeichnet durch** miteinander Verbinden der Bahnen und hieraus Ausschneiden der Artikel mit Hilfe eines Ultraschallschweißverfahrens.

5. Verfahren nach einem der Ansprüche 1 - 4, **gekennzeichnet durch** Ausbilden eines Aufhängebandes (21B - 21D) auf dem Artikel (18B - 18D), wenn die Bahnen (3, 4) miteinander verbunden werden und Ausschneiden fertiger Artikel.

6. Verfahren nach Anspruch 5, **gekennzeichnet durch** Ausbilden des Aufhängebandes (21B - 21C) in einem der Ränder des Artikels (18B - 18C), der sich in Maschinenrichtung längserstreckt; und Einlegen eines Verstärkungsstreifens (24C) zwischen den Bahnen (3, 4), so dass sich der Streifen im Bereich des Aufhängebandes eines jeden Artikels erstreckt.

7. Verfahren nach einem der vorhergehenden Ansprüche, **gekennzeichnet durch** Zuführen einer ersten Schicht (5, 6) in ein Ultraschallschweißgerät (26, 27) mit einer Geschwindigkeit, die größer ist als die Geschwindigkeit der Schicht (7, 8), an die die erste Schicht anzubringen ist, um so jede der ersten Schichten der Materialbahnen dauerhaft zu knittern; und miteinander Verbinden der Schichten mittels eines Musters aus einzelnen Befestigungspunkten, die sich über die gesamte Oberfläche der ersten Schicht erstrecken.

8. Verfahren nach einem der vorhergehenden Ansprüche, **gekennzeichnet durch** Zuführen einer dritten Bahn aus absorbierendem Material zwischen die zueinander hingerichteten glatten Schichten der ersten und zweiten Bahnen vor dem Verbinden der Bahnen miteinander.

9. Artikel zum Absorbieren von Blut und anderen Wundflüssigkeiten, wie beispielsweise eine Kompresse, ein Tuch oder ein chirurgisches Tuch, hergestellt durch das Verfahren nach Anspruch 1, wobei der Artikel zwei absorbierende erste Schichten (10, 12; 31, 36) aus einem Nonwoven-Material, die auf ihren jeweiligen gesamten Oberflächen dauerhaft geknittert sind, und zumindest eine glatte zweite Schicht (7, 8; 33) aus thermoplastischem Material beinhaltet, wobei die Schichten miteinander verbunden sind, jede erste Schicht mit einer glatten zweiten Schicht aus thermoplastischem Material durch ein Muster aus diskreten Befestigungspunkten verbunden ist, das sich über die gesamte Oberfläche der ersten Schicht erstreckt, wobei mindestens eine zweite Schicht zwischen die ersten Schichten platziert ist.

10. Artikel nach Anspruch 9, **dadurch gekennzeichnet, dass** der Artikel zwei glatte zweite Schichten (7, 8) beinhaltet.

11. Artikel nach Anspruch 9 oder 10, **dadurch gekennzeichnet, dass** der Artikel ein Aufhängeband (21B - 21D) beinhaltet, das einstückig mit dem Artikel (18B - 18D) ausgebildet ist.

12. Artikel nach einem der Ansprüche 9 - 11, **dadurch gekennzeichnet, dass** die erste Schicht (5, 6) aus einem zweilagigen Material (9, 10 bzw. 11, 12) besteht.

## Revendications

1. Procédé de fabrication de compresses, serviettes, draps chirurgicaux ou articles similaires destinés à absorber du sang ou d'autres fluides suintant d'une plaie, par l'association mutuelle de première et deuxième pièces de matériau (3, 4; 35, 36), parmi lesquelles l'une au moins de ces pièces comprend une première couche absorbante (5; 31) qui est plissée de façon permanente sur la totalité de sa surface et une deuxième couche (7; 33), lisse, en matériau thermoplastique, fixée à la première couche, et parmi lesquelles la deuxième pièce au moins comprend une première couche (6; 36) qui est plissée sur la totalité de sa surface, lesdites première et deuxième pièces étant associées avec les premières couches plissées (5, 6; 31, 36) tournées à l'opposé l'une de l'autre, les plis de la première couche respective étant réalisés et rendus permanents en fixant la première couche à une deuxième couche, lisse, de matériau thermoplastique, chaque première couche étant réunie avec une deuxième couche, lisse, de matériau thermoplastique au moyen d'un motif de points de fixation séparés qui s'étend sur toute la surface de la première couche, et par la réunion mutuelle des deux pièces de matériau (3, 4; 35, 36) et le découpage des articles (18; 40) à fabriquer dans les pièces ainsi réunies.

2. Procédé selon la revendication 1, caractérisé par l'utilisation de pièces de matériau (3, 4) qui comprennent chacune une première couche absorbante (5, 6) plissée de façon permanente sur la totalité de sa surface et une deuxième couche, lisse, de matériau thermoplastique (7, 8) qui est fixée à la première couche, la réunion des deux pièces le long des contours des articles (18) à fabriquer et le découpage des articles dans les pièces ainsi réunies dans les lignes de jonction.

3. Procédé selon la revendication 2, caractérisé par la réunion des deux pièces (3, 4) et le découpage des articles finis (18) dans les pièces en une seule opération.

4. Procédé selon la revendication 3, caractérisé par la réunion des deux pièces et le découpage des articles dans ces pièces à l'aide d'une opération de soudage par ultrasons.

5. Procédé selon l'une quelconque des revendications 1 à 4, caractérisé par la formation d'une sangle de suspension (21B-21D) sur l'article (18B-18D) lors de la réunion des pièces (3, 4) et le découpage des articles finis.

6. Procédé selon la revendication 5, caractérisé par la formation de la sangle de suspension (21B-21C) dans l'un des bords de l'article (18B-18C) qui s'étend longitudinalement dans la direction de la machine et la mise en place d'une bande de renfort (24C) entre les pièces (3, 4) de telle sorte que ladite bande s'étende dans la région de la sangle de suspension de chaque article.

7. Procédé selon l'une quelconque des précédentes revendications, caractérisé par l'amenée de la première couche (5, 6) à un dispositif (26, 27) de soudage par ultrasons à une vitesse supérieure à la vitesse de la couche (7, 8) à laquelle la première couche doit être fixée, de manière à plisser de façon permanente chacune des premières couches des pièces de matériau, et la réunion des couches au moyen d'un motif de points de fixation séparés qui s'étend sur toute la surface de la première couche.

8. Procédé selon l'une quelconque des précédentes revendications, caractérisé par l'amenée d'une troisième pièce de matériau absorbant entre les couches lisses se faisant face des première et deuxième pièces avant la réunion desdites pièces.

9. Article destiné à absorber du sang ou d'autres fluides suintant d'une plaie, comme une compresse, une serviette ou un drap chirurgical fabriqué selon le procédé de la revendication 1, l'article contenant deux premières couches absorbantes (10, 12; 31, 36) en matériau non tissé qui sont plissées de façon permanente sur la totalité de leurs surfaces respectives et au moins une deuxième couche (7, 8; 33), lisse, en matériau thermoplastique, lesdites couches étant réunies l'une à l'autre, chaque première couche étant réunie avec une deuxième couche, lisse, de matériau thermoplastique au moyen d'un motif de points de fixation séparés qui s'étend sur toute la surface de la première couche, la deuxième couche au nombre d'au moins une étant placée entre les premières couches.

10. Article selon la revendication 9, caractérisé en ce que ledit article contient deux secondes couches lisses (7, 8).

11. Article selon la revendication 9 ou 10, caractérisé en ce que ledit article contient une sangle de suspension (21B-21D) qui est formée d'un seul tenant avec l'article (18B-18D).

12. Article selon l'une quelconque des revendications 9 à 11, caractérisé en ce que la première couche (5, 6) est faite d'un matériau à deux couches (respectivement 9, 10 et 11, 12).
